# EUROPEAN PATENT SPECIFICATION

(11) **EP 4 159 134 B1**
(45) Date of publication and mention of the grant of the patent: **02.07.2025**
(21) Application number: 21812364.4
(22) Date of filing: 18.05.2021
(51) Int. Cl.: A61B 6/03, G01N 23/046

(54) **LOCKING ASSEMBLY FOR CT DETECTION DEVICE, AND CT DETECTION DEVICE**
VERRIEGELUNGSANORDNUNG FÜR CT-DETEKTIONSVORRICHTUNG UND CT-DETEKTIONSVORRICHTUNG
ENSEMBLE VERROUILLAGE POUR DISPOSITIF DE DÉTECTION CT, ET DISPOSITIF DE DÉTECTION CT

(30) Priority: 29.05.2020 CN 202010473343
(43) Date of publication of application: 05.04.2023
(73) Proprietor: Tsinghua University, Haidian District, Beijing 100084 (CN); Nuctech Company Limited, TongFang Building Shuangqinglu Haidian District Beijing 100084 (CN)
(72) Inventor: ZHANG, Li, Beijing 100084 (CN); HONG, Mingzhi, Beijing 100084 (CN); WANG, Zinan, Beijing 100084 (CN); HUANG, Qingping, Beijing 100084 (CN); ZHANG, Liguo, Beijing 100084 (CN)
(74) Representative: Gunzelmann, Rainer
(86) International application number: PCT/CN2021/094357
(87) International publication number: WO 2021/238720

(56) References cited:
- EP-A1- 3 342 348
- WO-A1-2018/190867
- CN-A- 105 326 526
- CN-A- 107 714 064
- CN-U- 203 576 528
- CN-U- 203 576 528
- CN-U- 208 931 962
- US-A1- 2017 258 428
- US-A1- 2020 155 111

## Description

### TECHNICAL FIELD

The present disclosure relates to a field of detection technology, and in particular to a locking assembly for a CT detection device and a CT detection device.

### BACKGROUND

In an existing CT detection device, a radiation source and a detector are mounted on a rotating frame. The rotating frame is mounted on a support stand through a slip ring bearing and is rotatable around a central axis of the rotating stand, thereby achieving a tomoscan. The rotating frame and components mounted thereon are collectively referred to as a CT imaging system.

The rotating frame is fixed on the support stand through the slip ring bearing, and driven to rotate by multiple motors. When the CT detection device is operating, a rotation movement of the CT imaging system is used to achieve the imaging. The CT imaging system has a complex constitution, a relatively large volume and a large mass, and a center of gravity and a center of rotation do not coincide, therefore, when the detection device is in maintenance, the CT imaging system may not be stopped at a position of arbitrary angle, and an auxiliary locking device is required to lock the CT imaging system to facilitate maintenance of the detection device. In addition, during a replacement of the slip ring bearing, in order to reduce a maintenance workload, after the slip ring bearing and the CT imaging system are separated, the CT imaging system is required to be kept in an original position, so that the CT imaging system may be recovered as soon as possible after a new slip ring bearing is mounted on the support stand. Therefore, it is necessary to design a locking mechanism having a CT imaging system.

CN203576528U discloses a CT (computed tomography) gantry component and a CT machine. The CT gantry component includes a rotating portion, a CT bearing and a bearing support frame, a rotor of the CT bearing is connected with the rotating portion, a stator of the CT bearing is connected with the bearing support frame, a plurality of first threaded holes matched with first screws are formed in the bearing support frame, the first screws can closely contact with the surface of the rotating portion through the first threaded holes, the axis of each first screw is provided with a through hole, a plurality of second threaded holes matched with second screws are formed in the rotating portion, in one-to-one correspondence to the first threaded holes and coaxial with the first threaded holes, and the second screws can penetrate the through holes and are screwed in the second threaded holes. By the aid of the technical scheme, when the CT bearing is damaged, the CT bearing can be conveniently replaced.

US2017/258428A1 discloses an arrangement including a stationary part of a gantry of a computed tomography scanner and a first rotating part of the gantry of the computed tomography scanner. The first rotating part and the stationary part are connectable to one another via a bearing assembly such that the first rotating part is arranged in a bearing position relative to the stationary part and is mounted via the bearing assembly such that it is rotatable about a system axis. The first rotating part and the stationary part are connectable to one another via a holding apparatus such that the first rotating part is arranged in a holding position relative to the stationary part independently of the bearing assembly. In both the bearing position and the holding position, a central opening of the first rotating part and a central opening of the stationary part are arranged about the system axis.

### SUMMARY

Accordingly, in the present disclosure, it is desired that a CT imaging system may be stopped at a position of arbitrary angle during a maintenance of the CT detection device. Moreover, during a replacement of a slip ring bearing, it is desirable that the CT imaging system is kept at an original position after an old slip ring bearing is detached, thereby simplifying the maintenance workload.

Accordingly, the present disclosure is intended to solve at least one of the above-mentioned problems.

The invention provides a device according to claim 1.

### BRIEF DESCRIPTION OF THE DRAWINGS

Other objectives and advantages of the present disclosure will be apparent from the following descriptions of the present disclosure with reference to the accompanying drawings, which may facilitate a comprehensive understanding of the present disclosure. In the drawings,
FIG. 1 shows a CT detection device according to an embodiment of the present disclosure;
FIG. 2 shows a rotating frame according to an embodiment of the present disclosure;
FIG. 3 shows a support stand according to an embodiment of the present disclosure;
FIG. 4 shows a first sleeve according to an embodiment of the present disclosure;
FIG. 5 shows a second sleeve according to an embodiment of the present disclosure;
FIG. 6 shows a partial cross-sectional view of a CT detection device according to an embodiment of the present disclosure, wherein the CT detection device is in a maintenance state; and
FIG. 7 shows a partial cross-sectional view of a CT detection device according to an embodiment of the present disclosure, wherein the CT detection device is in a state of replacing a slip ring bearing.

### DETAILED DESCRIPTION OF EMBODIMENTS

Technical solutions of the present disclosure will be further described in detail through the following embodiments in combination with the accompanying drawings. In the specification, the same or similar reference numerals indicate the same or similar components. The following descriptions of the embodiments of the present disclosure with reference to the accompanying drawings are intended to explain the general inventive concept of the present disclosure, and should not be construed as limiting the present disclosure.

In addition, in the following detailed descriptions, for ease of explanation, various specific details are set forth in order to provide a comprehensive understanding of the embodiments of the present disclosure. However, it is obvious that one or more embodiments may be implemented without these specific details. In other instances, commonly known structures and devices are shown in diagram to simplify the drawings.

In an embodiment of the present disclosure, during a maintenance of a CT detection device, two sides of a support stand are respectively provided with a cylindrical pin hole, and a circumference of a rotating frame are provided with several cylindrical pin holes. When the CT imaging system rotates to a suitable position, a cylindrical pin is used to pass through the cylindrical pin hole on the rotating frame and the cylindrical pin hole on the support stand at the same time, so that the rotating frame is fixed on a desired position by using the cylindrical pin. During a replacement of a slip ring bearing, two fixing blocks are mounted on the support stand, and other ends of the fixing blocks are fixed on the rotating frame.

Although the locking mechanism in the above-mentioned embodiment may achieve a required locking during the maintenance of the CT detection device and the replacement of the slip ring bearing, disadvantages of the above-mentioned solution lie in that a processing workload of the parts is large, a space occupied is large, and a cost is high. As a random accessory, the fixing block is required to be stored for a long time due to few using time, and a space occupied is large during storage due to its large volume.

Regarding at least one aspect of the above-mentioned defects, another embodiment of the present disclosure provides an improved solution. The present disclosure will be described in more detail below in combination with the accompanying drawings.

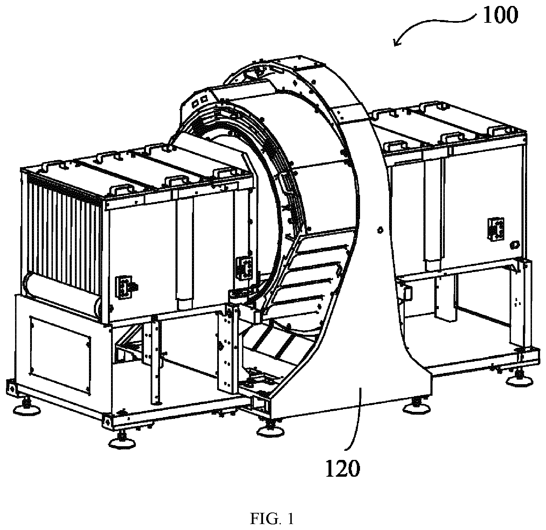

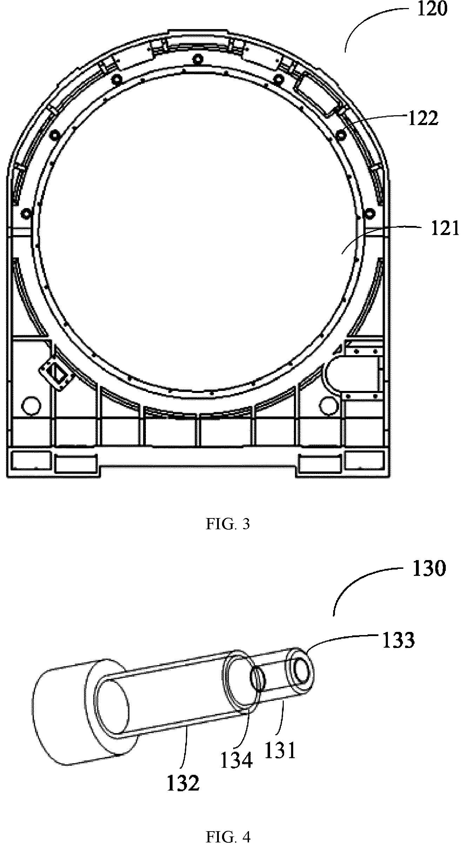

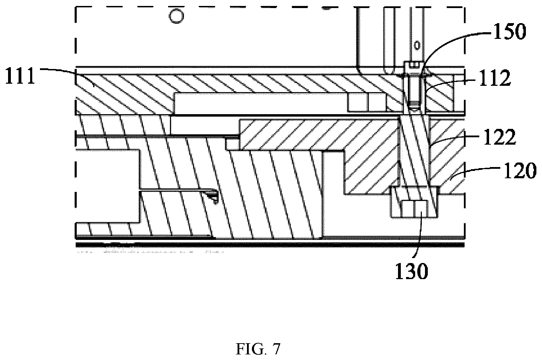

As shown in FIG. 1 to FIG. 4, the embodiment of the present disclosure provides a CT detection device 100, including a CT imaging system 110, a support stand 120 and a first sleeve 130. The CT imaging system 110 includes a radiation source, a detector and a rotating frame 111, and the radiation source and the detector are mounted on the rotating frame 111. A periphery of the rotating frame 111 is provided with at least one first hole 112. The rotating frame 111 is fixed on the support stand 120 through a slip ring bearing. A periphery of the support stand 120 is provided with at least one second hole 122 aligned with the at least one first hole 112. When the at least one first hole 112 is aligned with the at least one second hole 122, the first sleeve 130 is detachably engaged in the aligned first hole 112 and second hole 122. As shown in FIG. 4 and FIG. 7, the first sleeve 130 includes a first cooperating portion 133, and the first cooperating portion 133 cooperates with a first locking portion 150 to lock the rotating frame 111 during a replacement of the slip ring bearing. In this way, in the embodiment, a locking of the rotating frame is achieved by using less parts and structures during the replacement of the slip ring bearing, thereby simplifying the maintenance work.

In the embodiment, the radiation source provides a ray of, for example, one or more of an X-ray, a gamma (γ) ray, and a neutron ray, for detecting an object. The radiation source may be, for example, an accelerator, an isotope source, an X-ray machine or the like. In the embodiment, the detector is configured to detect the ray emitted from the radiation source.

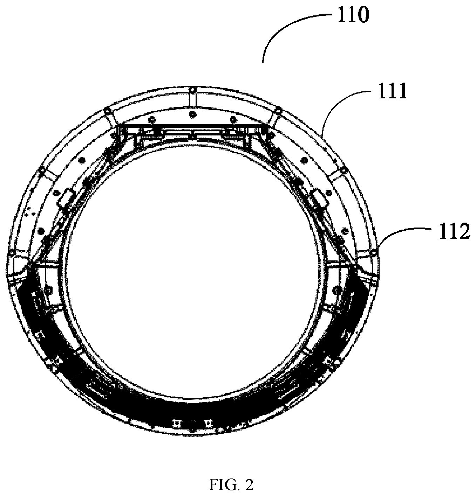

As shown in FIG. 2, the rotating frame 111 includes a plurality of first holes 112, and the plurality of first holes 112 are evenly distributed on the periphery of the rotating frame 111. As shown in FIG. 3, the support stand 120 includes an accommodating cavity 121, and the rotating frame 111 is mounted in the accommodating cavity 121. The support stand 120 includes a plurality of second holes 122, and the plurality of second holes 122 are evenly distributed on a periphery of the accommodating cavity 121. The examples shown in FIG. 2 and FIG. 3 are only illustrative, and quantities and positions of the first holes and the second holes may be adjusted by those skilled in the art as required.

As shown in FIG. 4, the first sleeve 130 includes a first cylindrical portion 131 and a second cylindrical portion 132. The first cylindrical portion 131 is engaged with the first hole 112, and the second cylindrical portion 132 is engaged with the second hole 122. In the embodiment, an outer surface of the second cylindrical portion 132 is provided with an external thread, and an inner surface of the second hole 122 is provided with an internal thread, so that the second cylindrical portion 132 cooperates with the second hole 122 in a threaded manner. In the embodiment, an outer surface of the first cylindrical portion 131 is provided with an external thread, and an inner surface of the first hole 112 is provided with an internal thread, so that the first cylindrical portion 131 cooperates with the first hole 112 in a threaded manner. An external thread may be provided on one or both of the two cylindrical portions to cooperate with an internal thread in a corresponding hole by those skilled in the art as required.

As shown in FIG. 4, a diameter of the first cylindrical portion 131 is smaller than a diameter of the second cylindrical portion 132. In the embodiment, the first sleeve 130 further includes a step 134 connecting the first sleeve portion 131 with the second sleeve portion 132.

In the embodiment, an inner surface of the first cylindrical portion 131 is provided with a thread to form a first cooperating portion 133, and the first cooperating portion 133 cooperates with a first locking portion 150 (such as a screw) to lock the rotating frame. In this way, a repositioning of the CT imaging system may be avoided after a new slip ring bearing is mounted, which facilitates subsequent operations. Moreover, in the solution of the present disclosure, the first locking portion having a simple structure and a small volume, such as a screw, is used, which reduces the workload of processing parts and a part space occupied by the locking assembly compared with a fixed block, thereby allowing a reduced dimension of the rotating frame. Those skilled in the art may understand that inner surfaces of both the first cylindrical portion 131 and the second cylindrical portion 132 may be provided with threads. Correspondingly, the first locking portion 150 cooperates with the threads on the inner surfaces of both the first cylindrical portion and the second cylindrical portion at the same time so as to achieve the locking. A specific providing manner may be selected by those skilled in the art as required.

In the embodiment, when the slip ring bearing is required to be replaced, the first sleeve 130 is mounted in the first hole 112 and the second hole 122, then the rotating frame 111 is rotated to a proper position. At this time, an end portion of the first sleeve 130 is attached to a surface of the rotating frame 111 which is located at a normal operation position, and the first locking portion 150 cooperates with the first cooperating portion 133 subsequently to achieve the locking. In this case, the end portion of the first sleeve is formed as a positioning surface to ensure that the position at which the rotating frame is located is the same as the position of its normal operation.

In the embodiment, when the CT detection device is required to be maintained, the first locking portion cooperates with the first cooperating portion, thereby fixing the rotating frame and allowing the rotating frame to be fixed at a position of arbitrary angle. In this way, two sets of locking assemblies used in two situations of replacing the slip ring bearing and maintaining the CT detection device are integrated into one set of locking assembly by the embodiment of the present disclosure, so as to achieve a locking requirement of the CT imaging system in different application scenarios with less parts and structures. Moreover, in the embodiments of the present disclosure, a locking function may be achieved conveniently and quickly, and the parts are small in volume and easy to be stored. Those skilled in the art may understand that another locking portion different from the first locking portion, such as a pin, may also be used when the CT detection device is required to be maintained.

In the embodiment, the CT detection device 100 further includes a second sleeve 140 configured to cooperate with the second hole 122. In the embodiment, an outer surface of the second sleeve 140 is provided with an external thread, and the inner surface of the second hole 122 is provided with an internal thread, so that the second sleeve 140 is mounted in the second hole 122 in a threaded manner.

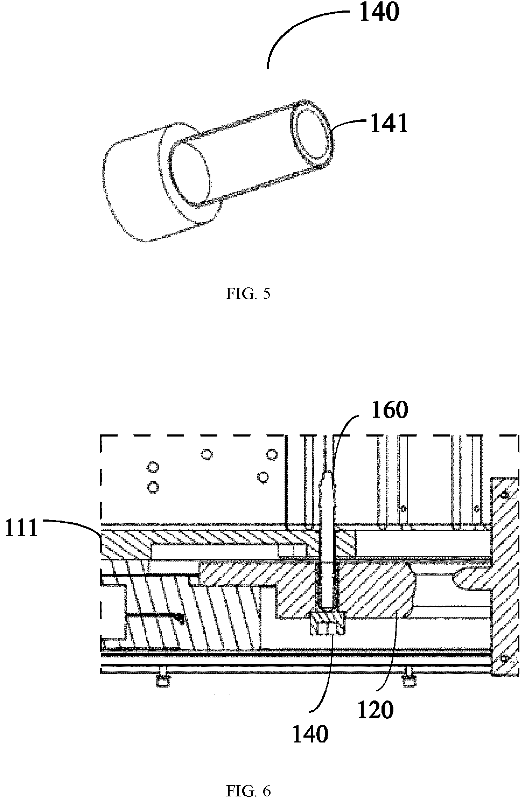

As shown in FIG. 5, the second sleeve 140 includes a second cooperating portion 141, and the second cooperating portion cooperates with a second locking portion 160 to lock the rotating frame 111 during the maintenance of the CT detection device 100. In the embodiment, a pin hole is formed inside the second sleeve 140, and the pin hole forms the second cooperating portion 141 to cooperate with the second locking portion 160 (such as a pin). As shown in FIG. 6, the pin is inserted into the first hole 112 and the pin hole inside the second sleeve 140, so that the rotating frame 111 is stopped at a desired position. In the embodiment, inner diameters of the pin hole and first hole 112 are substantially equal to an outer diameter of the pin. Those skilled in the art may understand that a threaded hole may be formed inside the second sleeve 140, and the threaded hole forms the second cooperating portion to cooperate with the second locking portion (such as a screw).

In the embodiment, the second sleeve 140 may be located in the second hole 122 when the CT detection device 100 operates normally. When the CT detection device 100 is required to be maintained, the second locking portion is inserted into the second cooperating portion to fix the rotating frame 111. As shown in FIG. 7, when the slip ring bearing is required to be replaced, the second sleeve 140 is removed from the second hole 122, then the first sleeve 130 is mounted in the second hole 122 and the first hole 112, and the first locking portion is mounted subsequently to lock the rotating frame 111. In this way, the locking requirement of the CT imaging system in different application scenarios is achieved by the embodiment of the present disclosure with less parts and structures, and the locking function may be achieved conveniently and quickly.

In the embodiments, both the first sleeve and the second sleeve may be formed by transforming screws, for example, by transforming finished hexagonal round heads, so that the cost is reduced and the workload of processing parts is reduced.

Those skilled in the art may understand that the embodiments described above are exemplary, and those skilled in the art may make improvements on them. The structures described in the various embodiments may be freely combined without structural or principle conflict.

Although the present disclosure has been described with reference to the accompanying drawings, the embodiments disclosed in the accompanying drawings are intended to illustrate preferred embodiments of the present disclosure, and should not be construed as limiting the present disclosure.It should be noted that the wording "include" does not exclude other elements or steps, and the wording "a" or "an" does not exclude a plurality. In addition, any reference numeral in the claims should not be construed as limiting the scope of the present disclosure.

## Claims

1. A CT detection device (100), comprising:
a CT imaging system (110), comprising a radiation source, a detector, and a rotating frame (111), wherein the radiation source and the detector are mounted on the rotating frame (111), and a periphery of the rotating frame (111) is provided with at least one first hole (112);
a support stand (120), wherein the rotating frame (111) is fixed on the support stand (120) through a slip ring bearing, and a periphery of the support stand (120) is provided with at least one second hole (122) aligned with the at least one first hole (112); and
a first sleeve (130) detachably engaged in the aligned first hole (112) and second hole (122), wherein the first sleeve (130) comprises a first cooperating portion (133), and the first cooperating portion (133) cooperates with a first locking portion (150) to lock the rotating frame (111) during a replacement of the slip ring bearing;
wherein the first sleeve (130) comprises a first cylindrical portion (131) engaged with the first hole (112) and a second cylindrical portion (132) engaged with the second hole (122);
wherein an outer surface of the first cylindrical portion (131) and an inner surface of the first hole (112) are provided with threads; and/or an outer surface of the second cylindrical portion (132) and an inner surface of the second hole (122) are provided with threads; and
wherein and the first cooperating portion (133) is formed as a thread on an inner surface of the first cylindrical portion (131) or as threads on inner surfaces of the first cylindrical portion (131) and the second cylindrical portion (132).

2. The CT detection device according to claim 1, wherein a diameter of the first cylindrical portion (131) is smaller than a diameter of the second cylindrical portion (132), and the first cylindrical portion (131) is connected with the second cylindrical portion (132) by a step (134); and
wherein a diameter of the first hole (112) is smaller than a diameter of the second hole (122).

3. The CT detection apparatus according to claim 1, wherein the first locking portion (150) is formed as a screw.

4. The CT detection device according to claim 1, wherein,
the at least one first hole (112) comprises a plurality of first holes (112) evenly distributed on the periphery of the rotating frame (111); and
the at least one second hole (122) comprises a plurality of second holes (122) evenly distributed on a periphery of an accommodating cavity (121) of the support stand (120), and the rotating frame (111) is mounted in the accommodating cavity (121).

5. The CT detection device according to claim 1, wherein an end surface of an end of the first sleeve (130) is a positioning surface.

6. The CT detection device according to any one of claims 1-5, further comprising a second sleeve (140) configured to be detachably engaged in the second hole (122) in a case that the first sleeve (130) is removed from the first hole (112) and the second hole (122), the second sleeve (140) further comprising a second cooperating portion (141), wherein the second cooperating portion (141) and the first hole (112) cooperate with a second locking portion (160) to lock the rotating frame (111) during a maintenance of the CT detection device (100);
wherein an outer surface of the second sleeve (140) and an inner surface of the second hole (122) comprise threads;
wherein the second cooperating portion (141) is formed as a pin hole inside the second sleeve (140), the second locking portion (160) is formed as a pin, and the pin is inserted into the first hole (112) and the pin hole inside the second sleeve (140); and
wherein inner diameters of the pin hole and the first hole (112) are substantially equal to an outer diameter of the pin.

7. The CT detection device according to claim 1, wherein the first locking portion (150) is formed as a pin, and the pin is inserted into the first hole (112) and the first cooperating portion (133) to lock the rotating frame (111) during a maintenance of the CT detection device (100).

## Patentansprüche

1. Ein CT-Detektionsgerät (100), umfassend:
ein CT-Bildgebungssystem (110), umfassend eine Strahlungsquelle, einen Detektor und einen Drehrahmen (111), wobei die Strahlungsquelle und der Detektor auf dem Drehrahmen (111) montiert sind und ein Umfang des Drehrahmens (111) mit mindestens einem ersten Loch (112) versehen ist;
einen Tragständer (120), wobei der Drehrahmen (111) über ein Schleifringslager an dem Tragständer (120) befestigt ist und ein Umfang des Tragständers (120) mit mindestens einem zweiten Loch (122) versehen ist, das mit dem mindestens einen ersten Loch (112) fluchtet; und
eine erste Hülse (130), lösbar in das ausgerichtete erste Loch (112) und das zweite Loch (122) eingesetzt, wobei die erste Hülse (130) einen ersten Zusammenwirkabschnitt (133) umfasst und der erste Zusammenwirkabschnitt (133) mit einem ersten Verriegelungsabschnitt (150) zusammenwirkt, um den Drehrahmen (111) während eines Austauschs des Schleifringslagers zu verriegeln;
wobei die erste Hülse (130) einen ersten zylindrischen Abschnitt (131), der mit dem ersten Loch (112) in Eingriff steht, und einen zweiten zylindrischen Abschnitt (132), der mit dem zweiten Loch (122) in Eingriff steht, umfasst;
wobei eine Außenfläche des ersten zylindrischen Abschnitts (131) und eine Innenfläche des ersten Lochs (112) Gewinde aufweisen und/oder eine Außenfläche des zweiten zylindrischen Abschnitts (132) und eine Innenfläche des zweiten Lochs (122) Gewinde aufweisen; und
wobei der erste Zusammenwirkabschnitt (133) als Gewinde an einer Innenfläche des ersten zylindrischen Abschnitts (131) oder als Gewinde an Innenflächen des ersten zylindrischen Abschnitts (131) und des zweiten zylindrischen Abschnitts (132) ausgebildet ist.

2. Das CT-Detektionsgerät nach Anspruch 1, wobei ein Durchmesser des ersten zylindrischen Abschnitts (131) kleiner ist als ein Durchmesser des zweiten zylindrischen Abschnitts (132) und der erste zylindrische Abschnitt (131) mit dem zweiten zylindrischen Abschnitt (132) durch eine Stufe (134) verbunden ist; und
wobei ein Durchmesser des ersten Lochs (112) kleiner ist als ein Durchmesser des zweiten Lochs (122).

3. Das CT-Detektionsgerät nach Anspruch 1, wobei der erste Verriegelungsabschnitt (150) als Schraube ausgebildet ist.

4. Das CT-Detektionsgerät nach Anspruch 1, wobei
das mindestens eine erste Loch (112) eine Mehrzahl erster Löcher (112) umfasst, die gleichmäßig am Umfang des Drehrahmens (111) verteilt sind; und
das mindestens eine zweite Loch (122) eine Mehrzahl zweiter Löcher (122) umfasst, die gleichmäßig am Umfang einer Aufnahmekavität (121) des Tragständers (120) verteilt sind, und der Drehrahmen (111) in der Aufnahmekavität (121) montiert ist.

5. Das CT-Detektionsgerät nach Anspruch 1, wobei eine Stirnfläche eines Endes der ersten Hülse (130) eine Positionierfläche ist.

6. Das CT-Detektionsgerät nach einem der Ansprüche 1-5, ferner umfassend eine zweite Hülse (140), die lösbar in das zweite Loch (122) eingesetzt wird, falls die erste Hülse (130) aus dem ersten Loch (112) und dem zweiten Loch (122) entfernt ist, wobei die zweite Hülse (140) einen zweiten Zusammenwirkabschnitt (141) umfasst, und wobei der zweite Zusammenwirkabschnitt (141) und das erste Loch (112) mit einem zweiten Verriegelungsabschnitt (160) zusammenwirken, um den Drehrahmen (111) während einer Wartung des CT-Detektionsgeräts (100) zu verriegeln;
wobei eine Außenfläche der zweiten Hülse (140) und eine Innenfläche des zweiten Lochs (122) Gewinde aufweisen;
wobei der zweite Zusammenwirkabschnitt (141) als Stiftloch innerhalb der zweiten Hülse (140) ausgebildet ist, der zweite Verriegelungsabschnitt (160) als Stift ausgebildet ist und der Stift in das erste Loch (112) und das Stiftloch innerhalb der zweiten Hülse (140) eingesetzt ist; und
wobei Innendurchmesser des Stiftlochs und des ersten Lochs (112) im Wesentlichen gleich einem Außendurchmesser des Stifts sind.

7. Das CT-Detektionsgerät nach Anspruch 1, wobei der erste Verriegelungsabschnitt (150) als Stift ausgebildet ist und der Stift in das erste Loch (112) und den ersten Zusammenwirkabschnitt (133) eingesetzt ist, um den Drehrahmen (111) während einer Wartung des CT-Detektionsgeräts (100) zu verriegeln.

## Revendications

1. Dispositif de détection TDM (100), comprenant:
un système d'imagerie TDM (110), comprenant une source de rayonnement, un détecteur et un cadre rotatif (111), dans lequel la source de rayonnement et le détecteur sont montés sur le cadre rotatif (111), et une périphérie du cadre rotatif (111) est pourvue d'au moins un premier trou (112);
un support (120), dans lequel le cadre rotatif (111) est fixé sur le support (120) par l'intermédiaire d'un roulement à bague collectrice, et une périphérie du support (120) est pourvue d'au moins un second trou (122) aligné avec au moins un premier trou (112); et
un premier manchon (130) inséré de manière amovible dans le premier trou (112) et le second trou (122) alignés, dans lequel le premier manchon (130) comprend une première partie de coopération (133), et la première partie de coopération (133) coopère avec une première partie de verrouillage (150) pour verrouiller le cadre rotatif (111) pendant le remplacement du roulement à bague collectrice;
dans lequel le premier manchon (130) comprend une première partie cylindrique (131) insérée dans le premier trou (112) et une seconde partie cylindrique (132) insérée dans le second trou (122);
dans lequel une surface extérieure de la première partie cylindrique (131) et une surface intérieure du premier trou (112) sont pourvues de filets; et/ou une surface extérieure de la seconde partie cylindrique (132) et une surface intérieure du deuxième trou (122) sont pourvues de filets; et
dans lequel et la première partie de coopération (133) est formée sous forme de filet sur une surface intérieure de la première partie cylindrique (131) ou sous forme de filets sur les surfaces intérieures de la première partie cylindrique (131) et de la seconde partie cylindrique (132).

2. Dispositif de détection TDM selon la revendication 1, dans lequel un diamètre de la première portion cylindrique (131) est inférieur à un diamètre de la seconde portion cylindrique (132), et la première partie cylindrique (131) est reliée à la seconde partie cylindrique (132) par un gradin (134); et
dans lequel le diamètre du premier trou (112) est inférieur au diamètre du second trou (122).

3. Dispositif de détection TDM selon la revendication 1, dans lequel la première partie de verrouillage (150) se présente sous la forme d'une vis.

4. Dispositif de détection TDM selon la revendication 1, dans lequel,
l'au moins un premier trou (112) comprend une pluralité de premiers trous (112) répartis à intervalles réguliers sur la périphérie du cadre rotatif (111); et
l'au moins un second trou (122) comprend une pluralité de seconds trous (122) répartis à intervalles réguliers sur la périphérie d'une cavité de logement (121) du support (120), et le cadre rotatif (111) est monté dans la cavité de logement (121).

5. Dispositif de détection TDM selon la revendication 1, dans lequel une surface terminale d'une extrémité du premier manchon (130) est une surface de positionnement.

6. Dispositif de détection TDM selon l'une quelconque des revendications 1 à 5, comprenant en outre un second manchon (140) conçu de manière à s'insérer de manière amovible dans le second trou (122) dans le cas où le premier manchon (130) serait retiré du premier trou (112) et du second trou (122), le second manchon (140) comprenant en outre une seconde partie de coopération (141), dans lequel la seconde partie de coopération (141) et le premier trou (112) coopèrent avec une seconde partie de verrouillage (160) pour verrouiller le cadre rotatif (111) pendant l'entretien du dispositif de détection TDM (100);
dans lequel une surface extérieure du second manchon (140) et une surface intérieure du second trou (122) comportent des filets;
dans lequel la seconde partie de coopération (141) se présente sous la forme d'un trou de tige à l'intérieur du second manchon (140), la seconde partie de verrouillage (160) se présente sous la forme d'une tige, et la tige est insérée dans le premier trou (112) et le trou de tige à l'intérieur du second manchon (140); et
dans lequel les diamètres intérieurs du trou de tige et du premier trou (112) sont sensiblement égaux au diamètre extérieur de la tige.

7. Dispositif de détection TDM selon la revendication 1, dans lequel la première partie de verrouillage (150) se présente sous la forme d'une tige, et la tige est insérée dans le premier trou (112) et la première partie de coopération (133) pour verrouiller le cadre rotatif (111) pendant l'entretien du dispositif de détection TDM (100).
